# EUROPEAN PATENT APPLICATION

(11) **EP 3 636 072 A1**
(43) Date of publication of application: **15.04.2020**
(21) Application number: 19827584.4
(22) Date of filing: 29.04.2019
(51) Int. Cl.: A01K 67/02

(54) **METHOD FOR CONSTRUCTING SLEEP DEPRIVATION-BASED YIN-DEFICIENCY FIRE-EXCESS TYPE ORAL ULCER ANIMAL MODEL**

(30) Priority: 29.08.2018 CN 201810996259
(71) Applicant: Sun Yat-Sen University, Guangzhou, Guangdong 510275 (CN)
(72) Inventor: YAO, Hongliang, Guangzhou, Guangdong 510275 (CN); SHEN, Pan, Guangzhou, Guangdong 510275 (CN); LI, Peibo, Guangzhou, Guangdong 510275 (CN); SU, Weiwei, Guangzhou, Guangdong 510275 (CN)
(74) Representative: Sun, Yiming
(86) International application number: PCT/CN2019/084920
(87) International publication number: WO 2020/042655

(57) **Abstract**

The present disclosure relates to a method for establishing an oral ulcer animal model with Yin-deficiency and fire-excess syndrome based on sleep deprivation, comprising the following steps of: burning buccal mucosa of a rat with phenol to cause a white injury, and obtaining the oral ulcer animal model after 24-48 h; placing the animal model in a sleep deprivation device for 2-7 days; wherein the device is composed of a mouse box in which a plurality of platforms with a diameter of 4-8 cm are arranged at an interval of 10-20 cm from each other; and water is filled among the platforms, and the platforms are about 0.1-2 cm higher than a water surface. The present disclosure overcomes the defects of single evaluation index, short model duration, and unconformity of etiology and clinic existed in the prior art.

## Description

### FIELD OF THE INVENTION

The present invention relates to a method for establishing an animal model in the field of medical research.

### BACKGROUND OF THE INVENTION

In traditional Chinese medicine, substances and functions with effects of moistening, condensing and inhibiting Yang heat belong to Yin. A Yin-deficiency syndrome refers to functional Yin-fluid deficiency and failure of Yin to control Yang, resulting in relative excitement of Yang and exhibiting dryness, heat, rise and movement, such as body temperature rise, weight loss, irritability, and other pathological states of functional deficiency excitement. At present, functional Yin-fluid deficiency caused by staying up late, appearance of dryness-heat, and the pathological state of functional deficiency excitement commonly exist. The pathophysiological research on Yin-deficiency and fire excess in modern medicine is mainly in endocrine system, immunity, energy metabolism, metabolic regulation imbalance and the like, thus having a preliminary explanation for an essence of a Yin-deficiency and fire-excess syndrome.

According to a theory of traditional Chinese medicine, causes of recurrent oral ulcer comprise Yin-deficiency and fire excess, flaring stomach fire, accumulation of heat in heart and spleen, spleen dysfunction due to dampness, Yang-deficiency of spleen and kidney, etc. However, oral ulcer caused by Yin-deficiency and fire excess is not recovered for a long time, with recurrent attacks accompanied by soreness and weakness of waist and knees, spermatorrhea and night sweat, steaming bone and hectic fever, red tongue with little coating, and other pathological states of functional deficiency excitement. The etiology of oral ulcer is complex, and the pathogenesis of oral ulcer is not yet clear. The modern medicine believes that the etiology of oral ulcer may be related to the following factors: inheritance, immunity, microorganism, hormone, psychological factors, deficiency of vitamin and trace element, and the like. Clinically, sleep insufficiency usually leads to recurrent oral ulcer.

The patent application 201710902646.2 discloses a method for establishing the oral ulcer animal model with Yin-deficiency and fire-excess syndrome. The application firstly provides a method for establishing the oral ulcer animal model with Yin-deficiency and fire-excess syndrome by subcutaneous injection of a triiodothyronine solution combined with phenol to corrode oral mucosa to cause ulcer. However, the method still has the following defects: its syndrome modeling idea is to simulate a clinical symptom of Yin-deficiency, which is quite different in etiology clinically; there are a small number of biochemical indexes detected, which are insufficiently related to the modern research on Yin-deficiency and fire excess and the complex etiology of oral ulcer; and meanwhile, the model does not last long enough.

Therefore, it is necessary to establish a disease-syndrome combination model highly related to the complex etiology of the oral ulcer with Yin-deficiency and fire-excess syndrome, the application of the model has more comprehensive biochemical index detection and more in-depth pharmacodynamic research on drugs for treating Yin-deficiency and fire-excess oral inflammation.

### SUMMARY OF THE INVENTION

In order to overcome the technical defects above, the present invention provides a method for establishing an oral ulcer animal model with Yin-deficiency and fire-excess syndrome caused by sleep insufficiency, and the method overcomes the defects of single evaluation index, short model duration, and unconformity of etiology and clinic existed in the prior art.

The technical solution adopted in the present invention is as follows: selecting a SD rat, exposing buccal mucosae on left and right sides, and placing the cotton balls soaked with phenol on the buccal mucosae on both sides of the SD rat to burn to cause white injuries. After 48 h, sleep deprivation is performed on the rat to obtain a target animal model. The method specifically comprises the following steps of:
I. preparing an oral ulcer animal model: burning buccal mucosa of a rat with phenol to cause a white injury, and obtaining the oral ulcer animal model after 24 h to 48 h; and
II. establishing a model with Yin-deficiency and fire-excess syndrome based on sleep-deprivation: placing the animal model in the step 1 in a sleep deprivation device for 2 days to 7 days, wherein the device is composed of a mouse box in which a plurality of platforms with a diameter of 4 cm to 8 cm are arranged at an interval of 10 cm to 20 cm from each other; and water is filled among the platforms, and the platforms are about 0.1 cm to 2 cm higher than a water surface.

The device has the function as follows: the rat is allowed to move on different platforms, when the rat falls asleep, the rat wakes up by rhythmically touching water with head bowed due to reduction of a muscle tension of a whole body, thus realizing sleep deprivation.

The platforms have a height of 5 cm to 10 cm, and the water has a temperature of 20°C to 25°C; and the rat is a SD rat with a weight of 150 g to 350 g.

The platforms preferably have a diameter of 6.3 cm, the platforms preferably have an interval of 15 cm from each other, and the platforms are preferably 1 cm higher than the water surface.

The oral ulcer animal model is preferably placed in the sleep deprivation device for 3 days.

The oral ulcer animal model with Yin-deficiency and fire-excess syndrome established by the present invention can be applied to research the effects of drugs for treating oral inflammation diseases caused by a pathological state of functional deficiency excitement due to chronic sleep insufficiency.

Compared with the existing animal model of the same type, the animal model of the present invention has the following advantages that: in the method of the present invention, sleep deprivation is performed on a model animal, which is similar to the pathological state of rising of deficiency fire and functional deficiency excitement caused by staying up late and sleep insufficiency. The animal model is closer to a clinical etiology and a symptom characteristic of a corresponding disease, and a symptom of the model lasts longer. The biochemical index detection of the animal model are closely related to Yin-deficiency and fire-excess syndrome and the complex etiology of oral ulcer by modern research. The present disclosure has an important application value for precise and in-depth research on effects of drugs for treating oral mucosa inflammation caused by the pathological state of rising of deficiency fire and functional deficiency excitement due to sleep insufficiency.

### BRIEF DESCRIPTION OF THE DRAWINGS

Fig. 1 illustrates a sleep deprivation device;
Fig. 2 illustrates a pathological observation result of an oral mucosa tissue of a rat under HE staining; and
Fig. 3 illustrates an area change of an oral ulcer of a rat.

### DETAILED DESCRIPTION OF THE EMBODIMENTS

The present invention is further described hereinafter with reference to the specific embodiments, but the present invention is not limited by the embodiments.

### 1. Experiment materials

(1) Experiment animals: 50 SPF adult male SD rats with a weight ranging from 200 g to 260 g, provided by Guangdong Medical Laboratory Animal Center, with a license number: SCXK (Yue) 2013-0002.
(2) Experiment reagents: isoflurane, phenol, formic acid, chloral hydrate, normal saline, 5-hydroxytryptamine (5-HT), γ-aminobutyric acid (GABA), and corticosterone (CORT); and detection kits of adrenocortical hormone (ACTH), immunoglobulin M (IgM), malondialdehyde (MDA), superoxide dismutase (SOD), tumor necrosis factor-α (TNF-α) and interleukin-1(β (IL-1β).
(3) Experiment instruments: a self-made sleep deprivation device, an anesthesia machine (MATRX), an electronic analytical balance (Sartorius), an infrared electronic thermometer (Kefu), a refrigerator (Hair), and a plastic slim tube (with a length of 6 cm and an inner diameter of about 4 mm).

Description for structure of the sleep deprivation device: the sleep deprivation device is a multi-platform water environment device, the device is composed of a mouse box (1) in which a plurality of platforms (2) with a diameter of 4 cm to 8 cm are arranged at an interval of 10 cm to 20 cm from each other; and water is filled among the platforms (3), and the platforms (2) are about 0.1 cm to 2 cm higher than a water surface.

### 2. Experiment method

(1) Experiment grouping: SD rats were randomly divided into three groups, comprising 20 rats in a normal group, 20 rats in an oral ulcer group (OU group) and 10 rats in a group of oral ulcer with Yin-deficiency and fire-excess syndrome (YU group). The experiment on the animals was started after the animals adapted to the environment for one week.
(2) Reagent preparation: phenol was firstly melted by slightly heat and then diluted to 95% with distilled water.
(3) The method for establishing a model: on the 1^{st} day of the experiment, the rats in the OU group and the YU group were anesthetized with isoflurane, their buccal mucosae on left and right sides were exposed, then a small cotton ball was placed at one end of a circular tube (with a length of 6 cm and an inner diameter of 4 mm) and flush with a tube orifice, 95% phenol was added dropwise until the cotton ball was just soaked, and the end of the circular tube at which cotton ball was placed was laid flat on the buccal mucosae on both sides of the rat to burn for 40 s finally to obtain a round white injury with a diameter of about 4 mm. After 48 h, observation was performed after anesthesia with isoflurane, and a digital camera was used to take a photo. 10 rats were randomly selected from the normal group and the OU group, anesthetized by intraperitoneal injection of 10% chloral hydrate (3 ml/kg), taken blood from abdominal aorta, and taken an oral mucosa tissue and a brain tissue, to measure relevant indexes. From the 3^{rd} day, sleep deprivation was performed on the rats in the YU group for 3 days; on the 6^{th} day, the rats in the YU group were observed and photographed, anesthetized with chloral hydrate, taken blood from abdominal aorta, and taken an oral mucosa tissue and a brain tissue, to measure relevant indexes.

### 3. Model evaluation

### 3.1 Evaluation for Yin-deficiency and fire-excess syndrome

### 3.1.1 General state: a change of a hair color, urination and defecation, emotional agitation, etc. were observed and recorded.

Weight: the rats were weighed with the electronic balance every morning.

Body temperature: a surface temperature of the rats was measured with the infrared electronic thermometer every afternoon.

### 3.1.2 Detection of indexes of a neuroendocrine and immune system

Neurotransmitter: brain tissues of the rats in each group were homogenized with 1.89% formic acid solution (tissue/1.89% formic acid solution: g/10 ml), and 5-HT and GABA in brain tissues and serum were measured; endocrine indexes: corticosterone (CORT) and adrenocorticotrophic hormone (ACTH) in serum were detected; and immunoinflammatory indexes: immunoglobulin M (IgM) and tumor necrosis factor-α (TNF-α) in serum were detected.

### 3.2 Evaluation for oral ulcer

After 48 h of burning, a size, a shape and a color of oral ulcer were observed, and photographed with a camera to calculate an ulcer area. Histopathological observation: after 24 h of establishing model, 3 rats in each group were randomly selected, anesthetized with 10% chloral hydrate, sacrificed by taking blood from abdominal aorta, and exposed the left and right buccal mucosae, to cut out a mucosal tissue of 6 mm×4 mm as deep as a submucosa. After washing with sterile normal saline, the mucosal tissue was fixed in 4% paraformaldehyde to obtain a sample. The sample was embedded with paraffin, and after making slices, a histopathological manifestation was observed. Detection of indexes of oxidative stress and inflammation: oral mucosa tissues of the rats in each group were taken, and weighed after drying with filter paper to prepare 10% tissue homogenate (tissue/normal saline: 1/9, m/m) .The 10% tissue homogenate was centrifuged (at 4°C and 5000 rpm for 10 min), a supernatant was taken and sub-packaged, and refrigerated at -80°C. Malondialdehyde (MDA), superoxide dismutase (SOD), tumor necrosis factor-α (TNF-α) and interleukin -1β (IL-1β) in the supernatant were measured according to kit description.

### 3.3 Date processing

Data was expressed by a mean value ± a standard deviation, SPSS 20.0 was used for one-way analysis of variance (ANOVA) and T test analysis. A P value less than 0.05 or 0.01was deemed as having a significant difference.

### 4. Experiment result

### 4.1 Result of Yin-deficiency and fire-excess syndrome:

4.1.1 General state: on the 6^{th} day, the rats in the group of oral ulcer with Yin-deficiency and fire-excess syndrome were observed, exhibiting the characteristics of dry and lusterless fur, an excited grasping reaction, and irritability. A body temperature of the rats in the group of oral ulcer with Yin-deficiency and fire-excess syndrome was significantly higher than that of the rats in the oral ulcer group and the normal group (P<0.01), reflecting characteristics of dysphoria with feverish sensation in chest, and hot and fire rise; and a body weight of the rats in the group of oral ulcer with Yin-deficiency and fire-excess syndrome was significantly lower than that of the rats in the oral ulcer group and the normal group (P<0.01), reflecting a characteristic of emaciation. While the rats in the ulcer group had no significant difference from the rats in the normal group, as shown in Table 1.

**Table 1 Changes of Body Weight and Body Temperature of Rats**

| Group | Body weight | Body temperature |
|---|---|---|
| Normal | 309.2±11.3 | 35.50±0.42 |
| OU | 295.4±13.9 | 35.51±0.47 |
| YU | 261.2±15.6** | 36.55±0.34** |

| | | |
|---|---|---|
| Note: compared with the normal group, *P<0.05; ** P<0.01 | | |

### 4.1.2 Detection of indexes of a neuroendocrine and immune system

### (1) Indexes of neurotransmitter:

On the 3^{rd} day, there was no significant difference in 5-HT and GABA in brain tissues and serum between the ulcer group and the normal group. However, on the 6^{th} day, after sleep deprivation, 5-HT and GABA in brain tissues and serum of the rats in the group of oral ulcer with Yin-deficiency and fire-excess syndrome were significantly higher than those of the rats in the normal group and the ulcer group, as shown in Tables 2 and 3.

**Table 2 Change of Neurotransmitter in Brain Tissue of Rats**

| Group | 5-HT (µg/g) | | GABA (µg/g) | |
|---|---|---|---|---|
| | 3^{rd} day | 6^{th} day | 3^{rd} day | 6^{th} day |
| Normal | 0.75±0.11 | 0.71±0.07 | 445.46±17.91 | 398.81±21.24 |
| OU | 0.73±0.06 | 0.71±0.08 | 430.21±18.50 | 396.41±24.95 |
| YU | | 0.81±0.07* | | 469.19±40.83** |

| | | | | |
|---|---|---|---|---|
| Note: compared with the normal group, *P<0.05; ** P<0.01 | | | | |

**Table 3 Change of Neurotransmitter in Serum of Rats**

| Group | 5-HT (ng/ml) | | GABA (ng/ml) | |
|---|---|---|---|---|
| | 3^{rd} day | 6^{th} day | 3^{rd} day | 6^{th} day |
| Normal | 1126±194 | 1115±190 | 9338±1087 | 7998±750 |
| OU | 1211±265 | 1147±138 | 9218±1651 | 8106±664 |
| YU | | 1604±351** | | 9614±2134** |

| | | | | |
|---|---|---|---|---|
| Note: compared with the normal group, *P<0.05; ** P<0.01 | | | | |

### (2) Indexes of endocrine:

On the 3^{rd} day, there was no significant difference in CORT and ACTH in serum between the ulcer group and the normal group. However, on the 6^{th} day, after sleep deprivation, CORT and ACTH in serum of the rats in the group of oral ulcer with Yin-deficiency and fire-excess syndrome were significantly higher than those of the rats in the normal group and the ulcer group (see Table 4), showing that sleep deprivation may activate a hypothalamus-pituitary-adrenal cortex axis, which was consistent with the modern research on Yin-deficiency.

**Table 4 Changes of CORT and ACTH in Serum of Rats**

| Group | CORT (ng/ml) | | ACTH (ng/L) | |
|---|---|---|---|---|
| | 3^{rd} day | 6^{th} day | 3^{rd} day | 6^{th} day |
| Normal | 148.0±18.7 | 150.6±35.7 | 63.91±16.24 | 71.60±6.16 |
| OU | 125.0±21.8 | 132.8±25.0 | 65.83±11.77 | 70.68±6.10 |
| YU | | 205.7±44.8** | | 96.88±14.09* |

| | | | | |
|---|---|---|---|---|
| Note: compared with the normal group, *P<0.05; ** P<0.01 | | | | |

### (3) Indexes of immune inflammation:

On the 3^{rd} day, there was no significant difference in IgM and TNF-α in serum between the ulcer group and the normal group. However, on the 6^{th} day, after sleep deprivation, IgM and TNF-α in serum of the rats in the group of oral ulcer with Yin-deficiency and fire-excess syndrome were significantly higher than those of the rats in the normal group and the ulcer group. Tables 6 and 7 showed that sleep deprivation slowed down the healing of oral ulcer by affecting body immunity. In an oral mucosa tissue, on the 3^{rd} day, there was significant difference in IL-1β and TNF-α between the ulcer group and the normal group, but on the 6^{th} day, there was no significant difference in IL-1β and TNF-α between the ulcer group and the normal group, while IL-1β and TNF-α of the rats in the group of oral ulcer with Yin-deficiency and fire-excess syndrome were still significantly higher than those of the rats in the normal group and the ulcer group (see Table 8), showing that an oral ulcer model with Yin-deficiency and fire-excess syndrome may last longer and was more stable.

**Table 5 Change of Indexes of Immune Inflammation in Serum of Rat s**

| Group | IgM (µg/L) | | TNF-α (ng/L) | |
|---|---|---|---|---|
| | 3^{rd} day | 6^{th} day | 3^{rd} day | 6^{th} day |
| Normal | 0.07±0.03 | 0.07±0.03 | 510.3±33.1 | 478.4±54.8 |
| OU | 0.08±0.03 | 0.08±0.03 | 561.2±59.5 | 530.7±70.3 |
| YU | | 0.12±0.04* | | 568.9±60.2* |

| | | | | |
|---|---|---|---|---|
| Note: compared with the normal group, *P<0.05; ** P<0.01 | | | | |

### 4.2 Results of oral ulcer

### 4.2.1 Ulcer and ulcer area

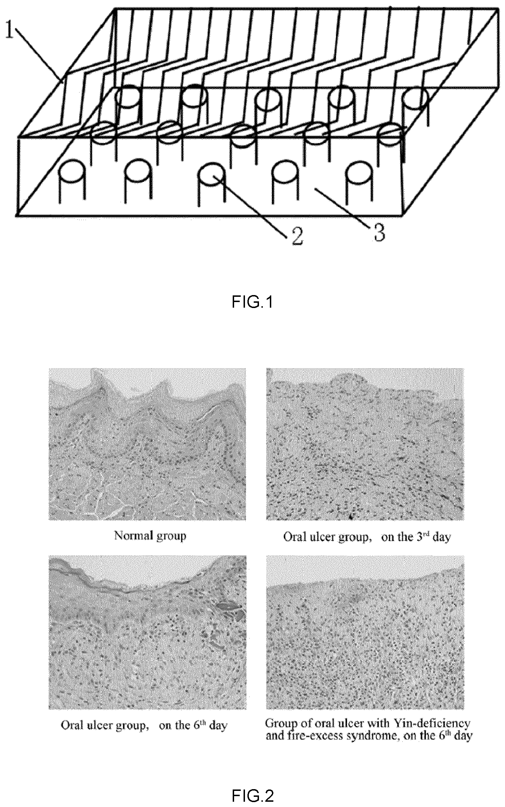

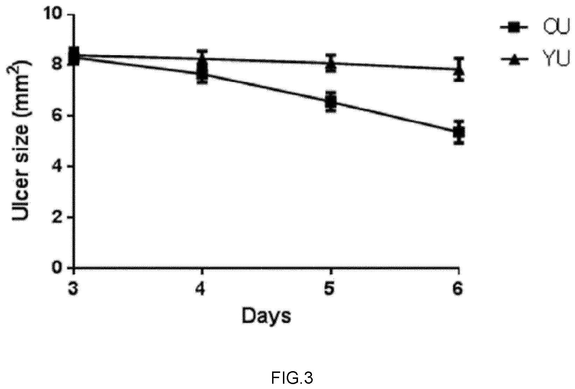

On the 3^{rd} day, that is, 48 h after burning with phenol, round ulcers with a diameter of about 4 mm appeared on buccal mucosae on both sides of the rats, with a necrotic tissue or a yellowish-white pseudomembrane covered on a surface. A pathological section result showed that an epithelium mucosae structure of the rats in the blank group was complete, and histological examination showed no abnormal change; and for the rats in the oral ulcer group, mucosal epithelium was missing, a granulation tissue could be seen in a lamina propria, and inflammatory cells were infiltered, as shown in Fig. 2. On the 6^{th} day, after sleep deprivation, an ulcer area of the rats in the group of oral ulcer with Yin-deficiency and fire-excess syndrome was significantly higher than that of the rats in the ulcer group (see Table 6), showing that an ulcer healing speed was significantly slowed down and a model may last longer, as shown in Fig. 3.

**Table 6 Change of Oral Ulcer Area in Rats**

| Group | Ulcer area on the 3^{rd} day | Ulcer area on the 6^{th} day |
|---|---|---|
| OU | 8.31±0.25 | 5.37±0.43 |
| YU | 8.40±0.28 | 7.84±0.43** |

| | | |
|---|---|---|
| Note: compared with the normal group, *P<0.05; ** P<0.01 | | |

### 4.2.2 Indexes of inflammation

In an oral mucosa tissue, on the 3^{rd} day, there was significant difference in IL-1β and TNF-α between the ulcer group and the normal group, but on the 6^{th} day, there was no significant difference in IL-1β and TNF-α between the ulcer group and the normal group, while IL-1β and TNF-α of the rats in the group of oral ulcer with Yin-deficiency and fire-excess syndrome were still significantly higher than those of the rats in the normal group and the ulcer group (see Table 7), showing that an oral ulcer model with Yin-deficiency and fire-excess syndrome may last longer and was more stable.

**Table 7 Change of Inflammation Indexes of Oral Mucosa Tissue of Rats**

| Group | TNF-α (pg/mgprot) | | IL-1β (pg/mgprot) | |
|---|---|---|---|---|
| | 3^{rd} day | 6^{th} day | 3^{rd} day | 6^{th} day |
| Normal | 6.93±0.67 | 5.82±2.77 | 1.25±0.34 | 0.90±0.13 |
| OU | 18.91±6.6** | 8.65±2.52 | 2.48±0.73 | 1.36±0.35 |
| YU | | 10.47±2.74** | | 2.39±0.53* |

| | | | | |
|---|---|---|---|---|
| Note: compared with the normal group, *P<0.05; ** P<0.01 | | | | |

### 4.2.3 Oxidative stress

On the 3^{rd} day, after formation of oral ulcer, MDA and SOD levels in an oral mucosa tissue were changed significantly. On the 6^{th} day, MDA and SOD levels of the rats in the ulcer group had no significant difference from those of the rats in the normal group, while MDA and SOD levels of the rats in the group of oral ulcer with Yin-deficiency and fire-excess syndrome were still significantly different from those of the rats in the normal group (see Table 8) showing that an oral ulcer model with Yin-deficiency and fire-excess syndrome constructed by sleep deprivation may increase ulcer duration.

**Table 8 Change of Oxidative Stress Indexes of Oral Mucosa Tissue of Rats**

| Group | MDA (nmol/mgprot) in tissue | | SOD (u/mgprot) in tissue | |
|---|---|---|---|---|
| | 3^{rd} day | 6^{th} day | 3^{rd} day | 6^{th} day |
| Normal | 0.90±0.22 | 0.78±0.16 | 149.76±17.17 | 150.24±18.96 |
| OU | 1.85±0.64* | 0.87±0.33 | 110.12±7.46** | 128.57±15.98 |
| YU | | 1.62±0.44* | | 116.11 ±8.32** |

| | | | | |
|---|---|---|---|---|
| Note: compared with the normal group, *P<0.05; ** P<0.01 | | | | |

## Claims

1. A method for establishing an oral ulcer animal model with Yin-deficiency and fire-excess syndrome based on sleep deprivation, comprising the following steps of:
(1) preparing an oral ulcer animal model: contacting phenol with buccal mucosa of a rat to form a white corrosion spot, and obtaining the oral ulcer animal model after 24 h to 48 h; and
(2) establishing a model with Yin-deficiency and fire-excess syndrome based on sleep deprivation: placing the animal model in the step 1 in a sleep deprivation device for 2 days to 7 days, wherein the device is composed of a mouse box in which a plurality of platforms with a diameter of 4 cm to 8 cm are arranged at an interval of 10 cm to 20 cm from each other; and water is filled among the platforms, and the platforms are about 0.1 cm to 2 cm higher than a water surface.

2. The method for establishing according to claim 1, wherein the platforms have a diameter of 6.3 cm.

3. The method for establishing according to claim 1, wherein the platforms have an interval of 15 cm from each other.

4. The method for establishing according to claim 1, wherein the platforms are 1 cm higher than the water surface.

5. The method for establishing according to claim 1, wherein the platforms have a height of 5 cm to 10 cm.

6. The method for establishing according to claim 1, wherein the water has a temperature of 20°C to 25°C.

7. The method for establishing according to claim 1, wherein the rat is a SD rat with a weight of 150 g to 350 g.

8. The method for establishing according to claim 1, wherein the oral ulcer animal model is placed in the sleep deprivation device for 3 days.
